Europäisches Patentamt

European Patent Office

Office européen des brevets

(19)

(11) Publication number: **0 041 373**
**A1**

## (12) EUROPEAN PATENT APPLICATION

(21) Application number: **81302368.6**

(22) Date of filing: **29.05.81**

(51) Int. Cl.³: **C 12 N 13/00**

(30) Priority: **30.05.80 US 154706**
**24.02.81 US 235471**

(43) Date of publication of application:
**09.12.81 Bulletin 81/49**

(84) Designated Contracting States:
**BE CH DE FR GB IT LI NL SE**

(71) Applicant: **PPG INDUSTRIES, INC.**
**One Gateway Center**
**Pittsburgh Pennsylvania 15222(US)**

(72) Inventor: **Carlin, William Worth**
**116 Fulton Place**
**Portland Texas 78374(US)**

(74) Representative: **Shipton, Gordon Owen et al,**
**W.P. THOMPSON & CO. Coopers Building Church Street**
**Liverpool L1 3AB(GB)**

(54) **Electrostimulation of microbial reactions.**

(57) A method of fermenting a substrate with a microorganism by forming a broth comprising the substrate and the microorganism, and forming a fermentation product therefrom characterised in that a fermentation stimulating electrical signal is imposed across the broth.

1.

## DESCRIPTION

## "ELECTROSTIMULATION OF MICROBIAL REACTIONS"

Microbe reactions, i.e., fermentation, are the biological conversion of a feedstock, i.e., a substrate, to a metabolite, i.e., a product, by the actions of microbes. Microbe reactions are characterized by the growth of the microbe, and the subsequent formation of a metabolite product. That is, substrate is used initially for the growth and maintenance of the microbes, and subsequently for both the growth and maintenance of the microbes and for the formation of product. Product formation is related to the concentration of substrate, the concentration of microbes, and the yield coefficients of product and microbes with respect to the substrate. Moreover, the concentration of microbes is a function of the specific growth rate of the microbes, and the yield coefficient of the microbes with respect to the substrate.

Fermentation reactions are typically slow reactions. That is, they require long residence time, i.e, high ratios of reaction medium volume per unit volume of production per unit time. They also have a long initiation time. That is, metabolite product cannot be formed in large quantities until high concentrations of microbe are present. Nutrient, i.e., substrate, is initially utilized primarily to grow and maintain microbes, and there-after it is utilized to a greater extent to form metabolite product.

Moreover, the reactions, involving microbial growth and division, are complex. For example, when the microbes are bacteria, cell growth and division is by fission, i.e., an individual cell will double in mass and content of cell constituents, and then split into two identical daughter cells. By way of contrast, yeasts are a class of microorganism that grow and divide by budding. That is, a bud will grow on an individual cell until it matches the size of the original cell, and then separate, leaving a bud scar. Fungi grow by chain enlongation and branching, i.e., with growth proceeding from the tip of the mycelium through the formation of septa between individual cells. Cell division may require anywhere from 15 minutes to an hour for bacterial growth, from 45 minutes to 2 hours for yeast growth, and from one to eight hours for fungi or mycelial growth.

The specific path of product synthesis, i.e., the reaction path for the formation of the metabolite product from the substrate or nutrient is not clearly understood for every fermentation reaction. However, it is believed to depend upon microbe growth and concentration, nutrient utilization, and metabolic controls.

It has now been found that the reaction rate of fermentation reactions, that is, the yield per unit time, unit volume, unit nutrient or substrate concentration, and unit microbe concentration is enhanced by the application of an alternating or pulsed high frequency electric field to the reaction medium of microbes, substrate, and nutrients.

As used herein, the terms "fermentation", "fermentation reactions", and "reactions utilizing fermentation techniques" include aerobic and anaerobic metabolic activity of a microbe or microbes in which chemical changes are brought about in an organic or inorganic substrate, and any process mediated by or involving microbes or microorganisms in which a product accrues.

As used herein, the terms "microbes", "microbe", microorganisms", and "micro-organism" include prokaryotes and eukaryotes. "Prokaryotes", as used herein, means unicellular microorganisms, including bacteria and unicellular blue-green algae. "Eukaryotes", as used herein, means multi-cellular microorganisms, including fungi, yeasts and actinomycetes. As used herein, the terms "microbes", "microbe", "microorganisms" and "microorganism" include immobilized microbes and systems and structures of immobilized microbes as well as non-immobilized microbes. As used herein, the terms "microbes", "microbe", "microorganisms", and "microorganism" include both naturally occurring strains and artificial recombinant strains.

Fermentation reactions, i.e., fungus, yeast, actinomycetes, and bacterial fermentation reactions are utilized industrially. Fermentation reactions are capable of wider industrial use based upon raw material availability and costs, by-product usage, and increased reaction rates.

According to the invention described herein, any or all of the reaction rate, the innoculum build up, or efficiency of conversion are increased by electrical stimulation. Electrical stimulation may be utilized to reduce the time required to carry out the reaction or to increase the productivity of a given unit of production, that is to increase the number of batches that can be fermented in a given reaction vessel.

By electrical stimulation is meant the application of a pulsed or alternating high frequency electric signal field across the broth whereby to pass an electrical current through the broth, e.g., by inserting electrodes in the broth, under conditions which increase the rate of the microbial reaction, while substantially avoiding significant destruction of the micro-organisms, e.g., fungi, bacteria, yeasts or actinomycetes, the reactants, e.g., the substrate, or the products.

It has been found that a frequency of from about 1 kilohertz to about 1000 kilohertz is particularly satisfactory in carrying out the method of this invention.

4.

The electrical stimulation electrical current may be an alternating current or pulsed direct current. It should have a frequency of from about 1 kilohertz to about 1000 kilohertz, although frequencies below about 10 megahertz may be used as well as frequencies above about 0.1 kilohertz with some increases in yield. However, care should be taken e.g. by using a frequency above about 0.1 kilohertz, to avoid large amounts of electrolysis occurring within the reaction medium at low frequencies.

The method of this invention may advantageously be carried out at various ranges of the electrolytic variables, i.e., electrode area, inter-electrode spacing, inter-electrode volume, current, current density, current per unit inter-electrode volume, current per unit broth volume, voltage, voltage per unit inter-electrode spacing, power per unit inter-electrode volume, power per unit broth volume, and frequency.

For example, the method of this invention has been carried out utilizing Saccharomyces cervisiae to ferment glucose at currents per unit interelectrode volume of from about $1 \times 10^{-3}$ milliamperes per cubic centimeter to about $30 \times 10^{-3}$ milliamperes per cubic centimeter of inter-electrode volume, at currents per unit broth volume of from about $1 \times 10^{-4}$ milliamperes per cubic centimeter to about $50 \times 10^{-4}$ milliamperes per cubic centimeter of broth, current densities of about $2 \times 10^{-2}$ milliamperes per square centimeter to $5 \times 10^{-1}$ milli-amperes per square centimeter of electrode area, voltage fluxes of 0.1 to 3 millivolts per centimeter of inter-electrode space, interelectrode power dissipations of $0.2 \times 10^{-7}$ watts per cubic centimeter to $6 \times 10^{-7}$ watts per cubic centimeter of interelectrode volume and broth power dissipations of $0.2 \times 10^{-8}$ watts per cubic centi-meter of broth volume to $8 \times 10^{-8}$ watts per cubic

centimeter of broth volume. Electrolytic variables, i.e. currents, voltages, and power dissipations, and products and quotients thereof, outside the above ranges may be utilized as long as care is taken, by avoiding simultaneous astomer of the ranges, to avoid destruction of the microbes.

According to one exemplification of the method herein contemplated, a reaction medium of a hexose, e.g., glucose, other nutrients, and a yeast, _Saccharomyces cerevisiae_ is provided. A pair of electrodes are spaced about 4 to about 7 centimeters from each other within the broth, and an electrical current at a current density of about 0.1 to about 0.3 milliamperes per square centimeter of electrode area is passed through the reaction medium at a frequency of about 100 kilohertz to about 1000 kilohertz. In this way, the yield of product per unit of substrate per unit time is increased by about 15 to about 20 percent compound with the result without electrical stimulation.

According to an alternative exemplification of the method of this invention, a reaction broth of a bacterium such as _Bacillus polymyta_ or _Bacillus licheniformis_, and glucose are prepared. The fermentation reaction is then carried out while an alternating current having a frequency of about 1 kilohertz to about 1000 kilohertz, an imposed voltage signal sufficient to provide a current of $1 \times 10^{-3}$ milliamperes per cubic centimeters of interelectrode volume and $10^{-1}$ to 5 milliampers per liter of solution is imposed across the broth. The resulting production rate of butane diol is increased by about 20 percent compared with the result without electrical stimulation.

The method of electrostimulated fermentation herein described is useful with single cell blue-green algae, bacteria, yeasts, and actinomycetes. Suitable yeasts are for example, baker's yeast and brewer's yeast, i.e.,

6.

0041373

single cell yeast of the Saccharomycoidicae group, as exemplified by Saccharomyces cerevisiae.

The method of electrostimulated fermentation is useful with various feedstocks. Exemplary are various carbohydrate substrates. By carbohydrates we mean for example polyhydroxy alkehydes, polyhydroxy ketones, and substances that yield polyhydroxy aldehydes or polyhydroxy ketones upon hydrolysis or saccharification. Exemplary carbohydrates are sugars, i.e. saccharides. The saccharides useful in the method of this invention may be monosaccharides, i.e., carbohydrates incapable of further hydrolysis, or polysaccharides, i.e, carbohydrates that yield monosaccharides upon hydrolysis or saccharification. Naturally occurring saccharides useful as substrates in the methods of this invention include heptoses, hexoses, pentoses, tetroses, triose, homo-polysaccharides thereof, and heteropolysaccharides thereof. Exemplary hexoses include glucose, fructose, mannose, galactose, and the fructose-glucose dissacharide, sucrose. Exemplary pentoses include arabinose, xylose, ribose, and apiose. Exemplary polysaccharides include sucrose, mentioned above, maltose, lactose, raffinose, starch, glycogen, cellulose, pectins, chitin, inulin, agar, hemicelluloses, plant gums and mucilages, and immunopolysaccharides. By carbohydrates we also mean sugar alcohols, e.g. sorbitol, mannitol, glactitol, or the inositols. Industrial sources of carbohydrate include by way of exemplification manure, cellulosic wastes, molasses, whey, sugar, grain starches, and byproduct carbohydrates. Suitable grain starches include by way of exemplification corn, corn stover, wheat, barley, straw, and bagasse.

Amino acids may be utilized as substrates for fermentation type reactions. They may be recovered as products of fermentation type reactions, or they may be intermediates, produced in one fermentation type reaction as a substrate for a subsequent fermentation type reaction. Exemplary alpha-amino acids include glycine, alanine, valine, leucine, isoleucine, serine, threonine, cysteine, cystine, methionine, phenylalanine, tyrosine, proline, tryptophan, lysine, arginine, histidine, aspartic acid, and glutamic acid. The above enumeration is intended to be exemplary and not exclusionary.

Other substrates which may be utilized are, for example, hydrocarbons, e.g., aliphatic hydrocarbons, carbocyclic hydrocarbons, and heterocyclic hydrocarbons. As used herein, hydrocarbons include substituted hydrocarbons, e.g. halogenated hydrocarbons, and hydrocarbons having one or more functional groups, e.g. alcohol, ketone, aldehyde, acid, ether, amine, amidine, N-alkyl amide, N,N-dialkyl amide, imidic ester, imide, aldimine, ketimine, thiol, thio ether, disulfide, thio acid, dithioacid, thio aldehyde, thio ketone, sulfonium, sulfoxide, sulfinic acid, sulfone, sulfonic acid, phosphite, phosphine, phosphonate, phosphate, orthocarbonate, carbonate, chloroformate, carbamate, carbamide (including urea), N-alkylurea, o-alkylurea, cyanate, isocyanate, carbodiimide, xanthate, thiocarbamate, thiocyanate, isothiocyanate, diazoate, or diazocyamide groups. In many cases the substrate is a dilute pollutant, and is degraded or metabolized to a non-polluting product.

The method of electrostimulated fermentation may be utilized with both aerobic and anaerobic fermentations.

Electrostimulated fermentation is useful in the industrial scale production of any product that can be produced by fermentation techniques, as described hereinabove. These products include the following, which enumeration is exemplary and not exclusionary: antibiotics; organic solvents, for example alcohols, e.g., butanol, ethanol, and amyl alcohols, ketones e.g. acetone;

gases, e.g. carbon dioxide and hydrogen;  beverages, e.g.
wines, beers and liquors;  foods, e.g. cheeses, fermented
milks, pickles, sauerkraut, soy sauce, yeast, vinegar, and
mushrooms;  flavoring agents e.g. monosodium glutamate;
organic acids and hydroxy acids e.g. lactic acid, acetic
acid, citric acid, gluconic acid, butyric acid, fumaric
acid, and itaconic acid;  glycerol; amino acids, e.g.
L-glutamic acid and L-lysine; steroids; organic transformations, including steroid, alkaloid, and antibiotic
transformations; yeasts, including food yeasts and
animal feed yeasts, legume innoculants; pesticides, e.g.
microbial and bacterial pesticides; vitamins and growth
stimulants, e.g. vitamin B-12 vitamin A, riboflavin and
gibberalkines;  enzymes including amylases, proteolytic
enzymes, pectinases, invernases, and cellulases, inter
alia;  fats; fatty acids; alcohols; fuels; and hydrocarbons.

The electrostimulation method herein contemplated
may also be used for the control or destruction of
deleterious substances, e.g., pollutants, and aqueous
dispersions, suspensions and solutions of hydrocarbons
or halocarbons, including polymers thereof.

While the electrostimulate fermentation method
described herein above is useful with various microbes
in various fermentation reactions, the method of this
invention may be exemplified by the Weizmann Process for
the bacterial fermentation of starch to yield n-butyl
alcohol, ethyl alcohol, and acetone;  the production of
ethyl alcohol by the use of Rhizopus formosaensis,
Saccharomyces cervisae, Saccharomyces uvarium, or
Aspergillus foetidus;  the production of acetic acid
utilizing Acetobactei alcoholophilus, Lactobacillus
plantarum, or Polyporus palustris; the production of
acetone utilizing Clostridiums; the production of butanol
utilizing Clostridiums; the production of glycerol utilizing Bacillus licheniformus or Saccharomyces rouxii; the production of acetic acid using Acetobactei pasteurianus; the production of wines using Saccharomyces chevalieri, Saccharomyces cervisiae, Saccharomyces roei, Saccharomyces vafer, Saccharomyces vini, Torulaspora florentina, Saccharomyces aceti, or Saccharomyces oxidans; the production of edible proteins utilizing C.haetomium cellalolytilum, Geotricum candidum, Candida utilis, Cellulmonas or Alcaligenes faecalis; the production of anthroquinones utilizing Chrysosporium merdarium, Helminthsporium cynadonitis, or Penicillium islandicum; the production of antibiotics utilizing Byssochlamys nivea, Fusarium equiseto, Gliocladium, Aspergillus astianus, Aspergillus sulphureus, Penicillium cyclopium, Penicillium martensi, Penicillium palitans, Penicillium puberulum, Aspergillus nidulans, Penicillium chryogenum, Penicillium notatum, acremonium strictum, Cephalasporium chrysogenum, Serratia rebidaea, Streptomyces lavendulae, Streptomyces clauligous, Streptomyces lipmanii, Acinetobacter calcoaceticus, Bacillus ceres, Bacillus licheniformis, or Bacillus subtilis; the production of L-arginine by Bacillus subtilis, Breribacterfum flurum, Corynebacterium glatamicum, or Protamino bacter thiaminophagus; the production of ascorbic acid by escherichia; the production of biotin by Corynebacterium primorioxydans, or Psuedomanus mutablis; the production of butanediols using Bacillus licheniformis, Bacillus polymyxa, or Klebsiella pneumoniae; the production of butyric acid using Butyrivibrio fibrisolvens; the production of caprylic acid using Ramibacterium alactolyticum; the production of carboxylic acid utilizing Sacchromycopsis lipolytica, or Sporolomyces odorus; the production of formic acid using Polyporus palustris; the production of fructose utilizing Bacillus megaterium, Pseudomanus boreopollis or Pseudomonas fluorescens; the production of gluconic acid using

10.

Aspergillus carbonarius, or Penicillum chrysogenum; the production of glucose utilizing Trichoderma longibrachiatum; the production of p-hydroxy benzaldehyde using Saccharomyces satic; the production of single cell proteins utilizing Arthrobacter petroleophagus, Arthrobacter rubellus, Arthrobacter, Aspergillus fumigatus, Cellulomonas cartelyticum, Corynebacterium fujiokemse, Kluyuromyces fragilis morehella crassipe, Mycobacterium cureatum, Mycoobacterium petroleophilum, or Nocardia neoopaca, and the production of xanthan gum using, e.g., Xanthamonas campestris. The method of electrostimulated fermentation may be used in the fermentation of substrates to obtain vitamins, antibiotics, and enzymes.

Electrostimulated fermentation also finds utility in microbial cellulose digestion, e.g., with Polyangium cellulosum; cleaning metallic surfaces, e.g., with Thiobacillus ferrooxidans or Thiobacillus thiooxidans; degradation of cellulose; degradation of cyanides in waste water, e.g., with Bacillus subtilis, Corynebacterium, or Nocardia rubropertincta; degradation of ethylene glycol with unidentified bacterium, ATCC 27042; degradation of chlorinated phenol fungicides; degradation of systemic fungicides, e.g., with Rhizopus japonicus; degradation of hydrocarbons, e.g., with Aspergillus versicolor, Brettanomyces petrophilum, Candida petrophilum, Candida tropicalis, Claclosporium resinae, Cunninghamella elegans, Eupenicillium zonatum, Saccharomycaporis lipulytica, or Torulopsis petrophilum; degradation of jet fuel, e.g., with Acremonium strietum, Alternaria alternata, Aspergillus jumigatas, or Cladosporium resinae; degradation of methanol, e.g., with Hansenula polymorpha; degradation of nitriles in waste water, e.g., with Alcaligenes visolactis, Nocardia rubroptincta, or Bacillus subtilis; degradation of petroleum, e.g. with Aspergillus aureohasidium, Candida parapsilosis, Candida

11.

tropicalis, Candida utilis, Cladosporium cladosporioices, Myrothecium verrucaria, Nocardia corallina, Nocardia globerula, Nocardia opain, Nocardia paraffinae, Nocardia rubra, Penicillium, Prototheca, Rhodotorula, Saccharomyces cervisiae, or Saccharomycopsis lipolytica; the degradation of phenol, e.g., with Gloeoporus dichrous, or Rhodutorula glutinis; the degradation of wood; phosphate removal in sewage treatment, e.g, with Chrysosporium pannorum, Geotrichum candidum, Mucar hiemalis, and Paecilomyces carneus; water pollution control, e.g., with Rhodotorula glutinis and Trichothecium roseum; and the production of dextrans by the fermentation of sucrose, e.g., with Leuconostoc mesenteroides and Betacoccus arabino sareus; and the production of immunopolysaccharides.

The following examples are illustrative:

### EXAMPLE I

Electrostimulated and conventional fermentations were carried out in a batch reactor.

The reaction broth was prepared by placing 1500 milliliters of distilled water in a sterilized 3 liter beaker. Carbohydrates, water and nutrients were added to the beaker in the following quantities:

| | |
|---|---|
| Glucose | 220 grams |
| NaCl | 3 grams |
| $(NH_4)_2SO_4$ | 6 grams |
| $K_2HPO_4$ | 2.4 grams |
| $KH_2PO_4$ | 0.4 grams |
| Distilled Water | to make 2 liters |

Thereafter, a 200 milliliter portion of the glucose solution was placed in a sterilized container and 14 grams of Fleischmann's Baker's Yeast, Saccharomyces cerevisae, was added thereto and stirred to form a slurry. The yeast-glucose slurry was divided into two equal

12.

portions of 100 milliliters each and put into two 1 liter sterilized resin kettles. To each of the divided yeast-glucose slurries were added 2.1 grams of corn meal, and 700 milliliters of the glucose solution.

The outlets of both reactors were connected to wet test meters to measure the gas produced. One reactor was run without electrical stimulation.

The other reactor had an electrode pair of two 4 square inch stainless steel electrodes, 4 centimeters apart. A signal generator was utilized to generate a 300 kilohertz, sine wave, voltage signal. A 50 milivolt signal caused a current of 1.5 milliamperes to flow.

Eight simultaneous, side-by-side runs were made, with one reactor having electrical stimulation and the other reactor being unstimulated.

The results shown in Table I, below, were obtained:

### TABLE I

### COMPARISON OF ELECTRO-STIMULATED FERMENTATION WITH CONVENTIONAL FERMENTATION

| Length of Run (hours) | Current (milli-amperes) | Moles of Ethanol-Stimulated (gel chro-matographic) | Moles of Ethanol-Unstimulated (gel chro-matographic) |
|---|---|---|---|
| 26.5 | 1.25 | 0.476 | 0.456 |
| 19 | 1.25 | 0.506 | 0.475 |
| 22 | 1.5 | 0.561 | 0.469 |
| 47.5 | 1.5 | 0.967 | 0.930 |
| 23.4 | 2.25 | 0.840 | 0.855 |
| 27 | 2.5 | 0.420 | 0.390 |
| 43.4 | 1.5 -6.1 | 1.14 | 1.16 |
| 25.5 | 2.25-6.5 | 0.713 | 0.707 |

### EXAMPLE II

Electrostimulated fermentation was carried out in a batch reactor to determine the effect of current and frequency.

The reaction broth was prepared by placing one liter of distilled water in a sterilized beaker. Carbohydrates, water and nutrients were added to the beaker in the following quantities:

| | |
|---|---|
| Sucrose | 100 grams/liter |
| NaCl | 1.5 grams/liter |
| $(NH_4)_2SO_4$ | 3.1 grams/liter |
| $K_2HPO_4$ | 1.2 grams/liter |
| $KH_2PO_4$ | 0.2 grams/liter |
| Distilled water | |

1.2 liters of the solution were placed in a 1.5 liter resin kettle.

Thereafter, 7.0 grams of Fleischmann's dried Baker's Yeast, saccharomyces cerevisiae, was poured on top of the reaction broth. The reactor was then closed, with gas venting through a water seal bubbler. The mixture was allowed to stand for 18 hours.

Thereafter, at 24 hour intervals, the slurry was removed from the reactor by aspiration and filtered to recover the yeast cake. The yeast cake was added to 1.0 liter of fresh reaction broth and returned to the kettle, which was sealed with a gas vent to a burette of 1 percent sulfuric acid and stirred for 5 minutes.

After two hours, four gas volume readings were taken at one half hour intervals, and used to calculate a base gas evolution rate, Ro. Thereafter, electrical stimulation was commenced. Four gas volume readings were taken at half hour intervals beginning one hour after electrical stimulation, and used to calculate a test gas evolution rate, $R_T$. For the fermentation with electrostimulation, $R_T$ is the gas evolution rate. For the fermentation without electrostimulation, $R_T$ is the gas evolution rate measured simultaneously with and calculated in the same way as the gas evolution rate for electrostimulation. After three hours of electrical stimulation, the current was turned off for 19 hours, and then reaction slurry removed as described above.

Each electrode was a nickel wire, 0.63 millimeters diameter, spiraled twice around a four inch (10 cm) long, 3 millimeter diameter glass rod. The electrodes were spaced 6.5 to 7.0 centimeters apart. Voltage was provided by a Dynascan 3010 function generator.

The effect of applied electrical field was as shown in Table II below:

### TABLE II

#### EFFECT OF APPLIED CURRENT (AT 100 KILOHERTZ) ON GAS EVOLUTION

| Current (milli-amperes) | $(R_T/Ro)$ (without current) | $(R_T/Ro)$ (with current) | $\dfrac{(R_T/Ro)\text{ with current}}{(R_T/Ro)\text{ without current}}$ |
|---|---|---|---|
| 0.15 | 1.05 | 1.15 | 1.10 |
| 0.15 | 0.92 | 1.02 | 1.11 |
| 1.5 | 1.01 | 1.13 | 1.12 |
| 1.5 | 0.98 | 1.17 | 1.19 |

The effect of frequency was as shown in Table III below:

### TABLE III

#### EFFECT OF FREQUENCY (AT 0.15 MILLIAMPERES) ON GAS EVOLUTION

| Frequency (kilohertz) | $(R_T/Ro)$ (without current) | $(R_T/Ro)$ (with current) | $\dfrac{(R_T/Ro)\text{ with current}}{(R_T/Ro)\text{ without current}}$ |
|---|---|---|---|
| 10 | 0.83 | 0.92 | 1.11 |
| 100 | 1.05 | 1.15 | 1.10 |
| 100 | 0.92 | 1.02 | 1.11 |

### EXAMPLE III

Fermentation of glucose with s.cervisiae was carried out with and without electrical stimulation.

15.

A broth was prepared containing 220 grams of glucose, 7.5 grams of S.cerevisae, 10 grams of $NH_4Cl$, 22 grams of $Na_2HPO_4 \times 7 H_2O$, 12 grams of $KH_2PO_4$, 1 gram of $MgSO_4$, and 0.04 grams of $CaCl_2$, and distilled water to make two liters.

The broth was divided into two portions and placed in sterilized resin kettles. Both kettles were stirred continuously with a magnetic stirrer. One kettle, intended for electrostimulation, had an electrode pair, each electrode was a 0.63 millimeter diameter nickel wire spiraled twice around a four inch (10 cm)long, 3 millimeter diameter, glass rod. The electrodes were spaced 6.5 to 7.0 millimeters apart.

The fermentation was carried out at 30 degrees Centigrade for 23.5 hours. A signal generator was utilized to generate a 300 kilohertz, 50 millivolt, sine wave signal. The results shown below were obtained:

S.CERVISIAE FERMENTATION
OF GLUCOSE

| | WITHOUT ELECTRICAL STIMULATION | WITH ELECTRICAL STIMULATION |
|---|---|---|
| FREQUENCY | --- | 300 kilohertz |
| CURRENT | --- | 1.5 milliamperes |
| INITIAL YEAST (GRAMS/LITER) | 3.4 | 3.5 |
| FINAL YEAST (GRAMS/LITER) | 8.8 | 9.3 |
| INITIAL GLUCOSE (GRAMS/LITER) | 108.9 | 106.3 |
| FINAL GLUCOSE (GRAMS/LITER) | 4.3 | <0.20 |
| INITIAL ALCOHOL (GRAMS/LITER) | 0.7 | 0.8 |
| FINAL ALCOHOL (GRAMS/LITER) | 43.0 | 54.4 |
| GRAMS OF YEAST/GRAMS OF GLUCOSE-HOUR | $2.18 \times 10^{-3}$ | $2.83 \times 10^{-3}$ |
| GRAMS OF ALCOHOL/GRAMS OF GLUCOSE-HOUR | $1.71 \times 10^{-2}$ | $2.18 \times 10^{-2}$ |
| GRAMS OF ALCOHOL/GRAMS OF YEAST (FINAL) | 4.87 | 5.72 |

CELL COUNT OF 200 SQUARES (three pipette samples per kettle)

|  | | |
|---|---|---|
| SAMPLE 1 | 660 | 865 |
| SAMPLE 2 | 739 | 895 |
| SAMPLE 3 | 632 | 804 |
| MEAN | 677 | 855 |
| RANGE | 107 | 91 |
| STANDARD DEVIATION | 63 | 53 |
| PERCENT RELATIVE STANDARD DEVIATION | 9.3 | 6.2 |
| DIFFERENCE IN MEANS | 178 | |

$$\sqrt{\frac{\sigma_1 2}{n_1} + \frac{\sigma_1 2}{n_2}} = $$

47

26 + 7% RELATIVE
(at $^-$1 standard deviation level)

($\sigma_1$ is the stranded deviation and $n_1$ is the number of replications).

The student's "t" test was applied to the cell counts. A Qt of 3.79 with a probability of 0.02 was obtained. That is, the probability that random errors would result in the 26% relative difference observed was less than 2 percent.

## EXAMPLE IV

A series of tests were conducted to compare electrostimulated fermentation with conventional fermentation. For each test a simple nutrient solution was prepared. A portion of the nutrient solution was withdrawn to make a yeast solution. The remainder of the nutrient solution was divided into two equal portions and placed into two identical laboratory fermenters. Both fermenters had an electrode pair. Fleischmann's Baker's Yeast, Saccharomyces cervisiae, was slurried in the remainder of the single nutrient solution. The nutrient solution, containing the yeast, was divided in half. Each half was placed into one of the two identical laboratory fermenters. A voltage

17.

signal was applied across one solution only of the pair. Solution samples were simultaneously taken from both solutions of the pair. Differences between the two fermentations of a pair were attributed to electrostimulation, while differences between sets of pairs of fermentations were attributed to conditions of the nutrient and innoculum solutions prior to commencing the tests.

For each fermentation a resin kettle fermenter was used. The resin kettle fermenter had an inside diameter of 10 centimeters, a depth of 15 centimeters, and was sealed on top. Each resin kettle fermenter had a sample probe, a pH probe, a mechanical stirrer, a sodium hydroxide inlet, a gas outlet, and an electrode pair. The electrodes were a pair of two inch (5 cm) by two inch (5 cm) stainless steel plate electrodes spaced two inches (5 cm) apart.

A glucose solution was prepared by adding in order:

| | |
|---|---|
| Glucose $\cdot$ $H_2O$ | 220 grams |
| $NH_4Cl$ | 10 grams |
| $Na_2HPO_4 \cdot 7H_2O$ | 22 grams |
| $KH_2PO_4$ | 12 gram |
| $MgSO_4$ | 1 gram |
| $CaCl_2$ | 0.04 gram |
| $H_2O$ | to make 2 liters |

The glucose solution was boiled, and the pH was adjusted to pH--5 with $H_3PO_4$.

A yeast slurry was prepared by withdrawing 200 milliliters of the glucose solution, and stirring 3.6 grams of Fleischmann's Baker's Yeast <u>Saccharomyces</u> <u>cervisiae</u> into the nutrient.

Each test was commenced by dividing the nutrient solution into two 800 milliliter portions and placing

18.

one portion of the nutrient solution into each resin kettle fermenter. Thereafter the slurry of yeast and nutrient was divided into two 100 milliliter portions. One portion of the yeast-nutrient slurry was added to each of the resin kettles.

The resin kettles were maintained at a temperature of 30 degrees Centigrade by immersion in a water bath. The pH was maintained between pH 4.7 and pH 5.2 by addition of aqueous NaOH. In each run a 300 kilohertz, 1.5 milliampere, 50 millivolt signal was applied across the electrode pair in one kettle. No signal was applied to the electrode pair in the other kettle.

The following results were obtained:

Set 1

| ELAPSED TIME | NO CURRENT | | CURRENT | |
|---|---|---|---|---|
| | Ethyl Alcohol (grms/ liter) | Glucose (grms/ liter) | Ethyl Alcohol (grms/ liter) | Glucose (grms/ liter) |
| (hours) | | | | |
| 1.25 | 1.17 | 98 | .72 | 100 |
| 3 | 1.87 | 96 | 1.96 | 98 |
| 6 | 5.28 | 78 | 5.72 | 79 |
| 25 | 31.8 | <0.25 | 40.4 | <0.25 |
| Final Yeast (grms/liter) | 2.54 | | 2.63 | |

Set 2

| ELAPSED TIME | NO CURRENT | | CURRENT | |
|---|---|---|---|---|
| | Ethyl Alcohol (grms/ liter) | Glucose (grms/ liter) | Ethyl Alcohol (grms/ liter) | Glucose (grms/ liter) |
| (hours) | | | | |
| 1 | 0.09 | 98 | 0.09 | 103 |
| 2 | 0.53 | 85 | 0.71 | 98 |
| 4.5 | 2.7 | 78 | 2.2 | 76 |
| 6.5 | 5.6 | 72 | 6.9 | 64 |
| 28 | 40.2 | <0.25 | 57.0 | <0.25 |
| Final Yeast (grms/liter) | 0.466 | | 0.504 | |

Set 3

| ELAPSED TIME | NO CURRENT | | CURRENT | |
|---|---|---|---|---|
| (hours) | Ethyl Alcohol (grms/ liter) | Glucose (grms/ liter) | Ethyl Alcohol (grms/ liter) | Glucose (grms/ liter) |
| 1 | 3.0 | 87 | 3.5 | 88 |
| 3 | 8.5 | 87 | 11.0 | 70 |
| 4.5 | 22.9 | 40 | 28.0 | 45 |
| 6 | 37.9 | 19 | 37.0 | 19 |
| 22.5 | 44.2 | ⟨0.25 | 48.7 | ⟨0.25 |
| Final Yeast (grms/liter) | 9.0 | | 9.8 | |

Set 4

| ELAPSED TIME | NO CURRENT | | CURRENT | |
|---|---|---|---|---|
| (hours) | Ethyl Alcohol (grms/ liter) | Glucose (grms/ liter) | Ethyl Alcohol (grms/ liter) | Glucose (grms/ liter) |
| 1 | 0.7 | 103 | 0.6 | 110 |
| 2 | 2.9 | 98 | 2.5 | 95 |
| 3 | 3.1 | 84 | 3.4 | 92 |
| 4 | 5.2 | 78 | 5.4 | 82 |
| 5 | 11.6 | 60 | 10.5 | 62 |
| 6 | 18.7 | 10.2 | 19.9 | 10.3 |
| 7 | 24.8 | .3 | 27.1 | .3 |
| 8 | 31.9 | --- | 32.0 | --- |
| 9 | 41.7 | --- | 51.0 | --- |
| 24 | 46.0 | ⟨0.25 | 56.0 | ⟨0.25 |
| Final Yeast (grms/liter) | 8.9 | | 9.6 | |

Set 5

| ELAPSED TIME | NO CURRENT | | CURRENT | |
|---|---|---|---|---|
| (hours) | Ethyl Alcohol (grms/liter) | Glucose (grms/liter) | Ethyl Alcohol (grms/liter) | Glucose (grms/liter) |
| 1 | 1.0 | 109 | 1.1 | 107 |
| 2 | 2.0 | 90 | 2.8 | 97 |
| 3 | 4.9 | 81 | 5.1 | 83 |
| 4 | 9.5 | 80 | 8.0 | 80 |
| 5 | 17.0 | 70 | 17.3 | 69 |
| 6 | 19.1 | 54 | 17.7 | 56 |
| 7 | 21.7 | 35 | 24.0 | 39 |
| 8 | 32.4 | 21 | 37.1 | 27 |
| 23.5 | 41.4 | 4 | 46.9 | <0.25 |
| Final Yeast (grms/liter) | 9.9 | | 10.5 | |

While the invention has been described with respect to certain exemplifications and embodiments, that is with respect to certain microbes, i.e., bacteria, actinomycetes, fungi, and yeasts, certain substrates, i.e., hydrocarbons, and carbohydrates, and certain products, it is not to be so limited, except as in the claims appended hereto.

-21-

# CLAIMS

1. A method of fermenting a substrate with a microorganism by forming a broth comprising the substrate and the microorganism, and forming a fermentation product therefrom characterised in that a fermentation stimulating electrical signal is imposed across the broth.

2. A method according to claim 1 characterised in that the microorganism is yeast, actinomycetes, bacteria, or unicellular blue-green algae.

3. A method according to claim 2 characterised in that the yeast is a <u>Saccharomyoideae</u> or a <u>Saccharomyces cervisiae</u>.

4. A method according to claim 1, 2 or 3 characterised in that the substrate is a carbohydrate, hydrocarbon or amino acid.

5. A method according to claim 4 characterised in that the carbohydrate is glucose, fructose or mannose.

6. A method according to claim 4 characterised in that the carbohydrate is a polysaccharide.

7. A method according to any of claims 1 to 6 characterised in that the electrical signal is an alternating current signal or a pulsed direct current signal.

8. A method according to any of claims 1 to 7 characterised in that the electrical signal has a frequency of 0.1 kilohertz to 10 megahertz.

9. A method according to any of claims 1 to 7 wherein the electrical signal has a frequency of 1 kilohertz to 1000 kilohertz.

10. A method according to any of claims 1 to 9 characterised in that the current per unit of interelectrode volume is from $1 \times 10^{-3}$ to $30 \times 10^3$ milliamperes per cubic centimeter.

11. A method according to any of claims 1 to 10 characterised in that the current per unit of broth is

from $1 \times 10^{-4}$ to $50 \times 10^{-4}$ milliamperes per cubic centimeter.

12. A method according to any of claims 1 to 11 characterised in that the current density is from $2 \times 10^{-2}$ to $50 \times 10^{-2}$ milliamperes per square centimeters.

13. A method according to any of claims 1 to 12 characterised in that the voltage flux is 0.1 to 3 millivolts per centimeter.

14. A method according to any of claims 1 to 13 characterised in that the interelectrode power dissipation is from $0.2 \times 10^{-7}$ to $6 \times 10^{-7}$ watts per cubic centimeter of interelectrode volume.

15. A method according to any of claims 1 to 14 characterised in that the broth power dissipation is from $0.2 \times 10^{-8}$ to $8 \times 10^{-8}$ watts per cubic centimeter of broth.

## DOCUMENTS CONSIDERED TO BE RELEVANT

| Category | Citation of document with indication, where appropriate, of relevant passages | Relevant to claim |
|---|---|---|
| X | AGRICULTURAL AND BIOLOGICAL CHEMISTRY, vol. 43, no.10, October 1979, Tokyo, JP, M. HONGO and M. IWAHARA: "Application of electroenergizing method to L-glutamic acid fermentation", pages 2075-2081 <br><br> * The whole document, in particular the abstract and page 2076, paragraph 2 * <br><br> -- | 1,2, 4-5,7, 12,13 |
| X | CHEMICAL ABSTRACTS, vol. 85, no. 25, December 20, 1976, page 401, abstract 190704h Columbus, Ohio, USA I. KREPIS et al. "Increase in biomass growth and in the physiological and biochemical activity of baker's yeasts Saccharomyces cerevisiae using electrophysical agents" <br><br> & ELEKTRON. OBRAB. MATER 1976 (4), 60-2 <br><br> *Abstract * <br><br> -- | 1-5,7 |
| X | DE - A - 2 841 933 (KABEL UND METALLWERKE GUTEHOFFNUNGSHUTTE) <br><br> * The whole document * <br><br> -- | 1-2,7 |
| X | DE - B - 2 052 548 (F.W. BRAUSS and A. VARGA) <br><br> * The whole document * <br><br> -- | 1-5, 7-12 |
| X | DE - C - 567 194 (F. SCHICK) <br> *                 ./. | 1-5,13 |

**CLASSIFICATION OF THE APPLICATION (Int. Cl.³)**

C 12 N 13/00

**TECHNICAL FIELDS SEARCHED (Int. Cl.³)**

C 12 N 13/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 05-08-1981 | GALLIGANI |

# DOCUMENTS CONSIDERED TO BE RELEVANT

| Category | Citation of document with indication, where appropriate, of relevant passages | Relevant to claim | CLASSIFICATION OF THE APPLICATION (Int. Cl.³) |
|---|---|---|---|
| | * Whole document * | | |
| | -- | | |
| X | DE - C - 572 960 (F. SCHICK) <br> * The whole document * | 1-5,13 | |
| | -- | | |
| X | CHEMICAL ABSTRACTS, vol. 77, no. 7, August 14, 1972, page 347, abstract 46742m <br> Columbus. Ohio. USA <br> & JP - A - 72 14745 (AJINOMOTO CO. INC) ( 10-05-1972) <br> * Whole abstract * | 1,2,4- 5,7, 13 | TECHNICAL FIELDS SEARCHED (Int. Cl.³) |
| | -- | | |
| X | CHEMICAL ABSTRACTS , vol. 75, no. 23, December 6, 1971, <br> pages 211-212, abstract 139401y <br> Columbus, Ohio, USA <br> & JP - A - 71 27712 (AJINOMOTO CO. INC.) (11-08-1971) <br> * Whole abstract * | 1,2,4, 5,7, 13 | |
| | -- | | |
| X | FR - A - 1 057 842 (H. KLIEWE, G. NEIDL) <br> * The whole document, in parti- cular: page 1, paragraph 3; page 4, summary * | 1,2,4, 5,7-12 | |
| | -- | | |
| X | DD - A - 74 753 (H. SCHROEDER) <br> * The whole document * | 1,2,4- 7,13 | |
| | --        ./. | | |

**0041373**

European Patent
Office

**EUROPEAN SEARCH REPORT**

Application number

EP 81 30 2368

| Category | Citation of document with indication, where appropriate, of relevant passages | Relevant to claim |
|---|---|---|
| X | FR - A - 843 994 (J.R.E SANDSTROM * Page 3, summary * ---- | 1-7 |

**DOCUMENTS CONSIDERED TO BE RELEVANT**

CLASSIFICATION OF THE
APPLICATION (Int. Cl.³)

TECHNICAL FIELDS
SEARCHED (Int. Cl.³)